# EUROPÄISCHE PATENTSCHRIFT

(11) **EP 0 519 902 B1**
(45) Veröffentlichungstag und Bekanntmachung des Hinweises auf die Patenterteilung: **11.09.1996**
(21) Anmeldenummer: 92890146.1
(22) Anmeldetag: 16.06.1992
(51) Int. Cl.: C12N 9/64

(54) **Verfahren zur Herstellung von aktiviertem Protein C**
Method of preparing activated protein C
Procédé pour la production de la protéine C activée

(30) Priorität: 20.06.1991 AT 1238/91
(43) Veröffentlichungstag der Anmeldung: 23.12.1992
(73) Patentinhaber: IMMUNO Aktiengesellschaft, A-1221 Wien (AT)
(72) Erfinder: Philapitsch, Anton, A-2490 Ebenfurt (AT); Schwarz, Hans Peter, Doz. Dr., A-1180 Wien (AT)
(74) Vertreter: Wolfram, Gustav, Dipl.-Ing.

(56) Entgegenhaltungen:
- EP-A- 0 416 890

## Beschreibung

Die Erfindung betrifft ein Verfahren zur Herstellung von aktiviertem Protein C, wobei Protein C mit einer Protease behandelt wird.

Protein C ist ein Vitamin-K-abhängiges Glycoprotein, das in der Leber synthetisiert wird und im Plasma in einer Konzentration von 4 µg/ml als inaktives Zymogen zirkuliert. Es wird an der Gefäßoberfläche (Endothel) durch den Thrombin-Thrombomodulinkomplex in die aktive Serinprotease (aktiviertes Protein C) übergeführt. Protein C kann auch durch einige nicht-physiologische Enzyme, wie "snake-venom"-Faktor (Protac C), aktiviert werden. Es ist bekannt, daß aktiviertes Protein C durch die Inaktivierung von Plasminogenaktivator-Inhibitor und einen noch unbekannten Protein-S-abhängigen Mechanismus fibrinolytische Eigenschaften besitzt. Es wirkt auch antikoagulatorisch, weil es den Faktor Va, den Kofaktor für die Faktor Xa-induzierte Prothrombinaktivierung (Thrombinbildung) und den Faktor VIIIa, den Kofaktor für die Faktor IXa-induzierte Faktor X-Aktivierung, durch Proteolyse abbaut.

Die Aktivierung von Protein C in vivo stellt eine negative Feedback-Reaktion der Thrombingenerierung dar. Um optimale biologische Aktivität zu entfalten, ist ein Kofaktor (Protein S) notwendig.

Die Aktivierung von plasmatischem Protein C ex vivo ist bekannt und wird durch Behandeln mit Aktivatoren, wie Thrombin, ThrombinThrombin-Thrombomodulin-Komplex oder Schlangengift vorgenommen.

In der EP-A - 0 287 028 wird Protein C durch Zugabe des ThrombinThrombin-Thrombomodulin-Komplexes aktiviert. Die Reaktion wird durch Inhibition des Thrombins mit Antithrombin III gestoppt, worauf das aktivierte Protein C affinitätschromatographisch gereinigt wird.

In Thrombosis research 59, 27-35 (1990) wird die Aktivierung des Protein C durch Kontakt mit einem gelösten oder immobilisierten Thrombin oder durch Recalcifizierung von einer Prothrombinkomplex-Präparation beschrieben. Das verwendete Thrombin ist von bovinem Ursprung, was ein Risiko der Kontamination des Produktes mit bovinen Substanzen mit sich bringt. Dieses Aktivierungsverfahren hat weiters den Nachteil, daß das aktivierte Protein C mit Thrombinspuren verunreinigt ist, weshalb eine reihe von affinitätschromatographischen reinigungsschritten im Anschluß an die Aktivierung notwendig wird.

Die Erfindung stellt sich die Aufgabe, ein verbessertes Verfahren zur Aktivierung von Protein C zur Verfügung zu stellen, das die oben genannten Nachteile nicht aufweist.

Diese Aufgabe wird bei einem Verfahren der eingangs erwähnten Art erfindungsgemäß durch Behandeln von Protein C mit Plasmin gelöst.

Es hat sich gezeigt, daß Plasmin, das Schlüsselenzym der Fibrinolyse, die Aktivierung des Protein C rasch und in einfacher Weise bewirkt, so daß ein völlig neuartiges Aktivierungsverfahren möglich wird.

Die Aktivierung des Protein C erfolgt vorteilhafterweise bei einem Protein C/Plasmin-Verhältnis (µg/CU) zwischen 80 und 40.000, vorzugsweise zwischen 4.000 und 40.000. Die Aktivierung wird am besten bei Temperaturen um 37°C vorgenommen. Humanes Plasmin ist leicht aus humanem Plasminogen herstellbar oder kommerziell erhältlich (z.B. Fa. Kabi).

Die erfindungsgemäße Aktivierung erfolgt rasch, d.h. unter Umständen innerhalb weniger Minuten, weshalb in manchen Fällen ein Zusatz von Calciumionen (5 bis 50 mmol/l) zur Verzögerung der reaktion vorteilhaft sein kann.

Die Aktivierung von Protein C mit Plasmin hat im Gegensatz zu Thrombin den Vorteil, daß Spuren des Aktivators in einem Protein C-Präparat dessen antikoagulative bzw. fibrinolytische Wirkung noch verstärken.

Da Plasmin als Protease insbesondere bei hohen Plasmin-Konzentrationen aktiviertes Protein C weiter abbauen kann, ist es vorteilhaft, die reaktion nach einer relativ kurzen Inkubationsdauer durch den Zusatz eines Plasmininhibitors, wie Antithrombin III oder Antithrombin III-Heparin-Komplex, zu stoppen. Die Reaktion kann beispielsweise bei Erreichen eines gewünschten Aktivierungsgrades (etwa bei einer Aktivität von mindestens 0,15 dE/min S 2366) gestoppt werden.

Ein durch Plasminbehandlung aktiviertes Protein C unterscheidet sich in seiner Aktivität oder nach SDS-PAGE-Analyse nicht von einem konventionell mit Thrombin aktivierten Protein C.

Nachfolgend wird die Erfindung noch näher beschrieben.

### Gewinnung von Protein C

Hochreines Protein C wurde aus einer rohen Protein C-Fraktion gewonnen, die aus kommerziell erhältlichem Prothrombinkomplex-Konzentrat hergestellt wurde. Die Reinigung erfolgte affinitätschromatographisch mittels monoklonaler Antikörper. Monoklonale Anti-Protein C-Antikörper wurden wie folgt hergestellt:

BALB/C-Mäuse wurden mit 100 µg menschlichem Protein C in zweiwöchigen Abständen durch intraperitoneale Injektion immunisiert. Nach sechs Wochen wurden noch einmal 50 µg des menschlichen Protein C injiziert und drei Tage später die Fusion vorgenommen. Die Myelomzellinie (P3-X-63-AG8-653, 1,5 x 10⁷ Zellen) wurde vermischt mit 1,7 x 10⁸ Milzzellen von einer Maus, und die Fusion nach modifizierter Köhler & Milestein-Methode unter Verwendung von PEG 1500 durchgeführt (Köhler G., Milestein C., Nature 256(1975)495-497).

Positive Klone, getestet mittels eines ELISA, wurden zweimal subgeklont. Aszitesproduktion erfolgte durch Injection von 5 x 10⁶ Hybridomzellen je BALB/C-Maus zwei Wochen nach Pristan-Behandlung.

Das Immunglobulin wurde aus Aszites durch Ammoniumsulfatpräzipitation und anschließende Chromatographie mittels QAE-Sephadex und darauffolgend Chromatographie an Sephadex G200 gereinigt. Um das Risiko der Übertragung muriner Viren zu reduzieren, wurde der Antikörper vor der Immobilisierung noch einem Virusinaktivierungsschritt unterworfen. Die so erhaltenen monoklonalen Antikörper gegen Protein C wurden an CNBR-Sepharose 4B (Pharmacia) gekoppelt. Für die Reinigung des Protein C mittels Affinitätschromatographie wurden folgende Puffer verwendet:

Als Absorptionspuffer: 20 mmol Tris, 2 mmol EDTA, 0,25 mol NaCl und 5 mmol Banzamidin;
als Waschpuffer wurde verwendet: 20 mmol Tris, 1 mol NaCl, 2 mmol Benzamidin, 2 mmol EDTA; der pH-Wert betrug 7,4; als Elutionspuffer wurde verwendet: 3 mol NaSCN, 20 mmol Tris, 1 mol NaCl, 0,5 mmol Benzamidin, 2 mmol EDTA.

Im einzelnen: Das Prothrombinkonplex-Konzentrat wurde im Absorptionspuffer aufgelöst, wobei etwa 10 g des Prothrombinkomplex-Konzentrates für eine 20 ml monoklonale Antikörpersäule zur Verwendung kamen. Anschließend wurde das aufgelöste Prothrombinkomplex-Konzentrat filtriert, bei 20.000 rpm 15 min lang zentrifugiert und durch ein Konzentrat filtriert, bei 20.000 rpm 15 min lang zentrifugiert und durch ein 0,8 µm Filter sterilfiltriert. Das sterilfiltrierte und gelöste Prothrombinkomplex-Konzentrat wurde auf die Säule aufgetragen mit einer Flußrate von 10 ml/h. Anschließend wurde die Säule mit dem Waschpuffer proteinfrei gewaschen und schließlich das gebundene Protein C mit dem Elutionspuffer bei einer Flußrate von 5 ml/h eluiert und die Fraktionen gesammelt. Das eluierte Protein C wurde gegen einen Puffer (0,2 mol/l Tris, 0,15 mol Glycin und 1 mmol EDTA, pH 8,3) dialysiert. Der Protein C-Gehalt wurde antigenmäßig mittels der Methode nach Laurell und aktivitätsmäßig nach Protac-Aktivierung bestimmt.

Das erhaltene Protein C-Eluat wurde in folgender Weise zu einer pharmazeutisch applizierbaren Präparation fertiggestellt:

Das Eluat wurde zuerst einem Ultrafiltrations- und einem Diafiltrationsschritt unterworfen. Für die Diafiltration wurde ein Puffer mit einem pH-Wert von 7,4 verwendet, der pro Liter 150 mMol NaCl und 15 mMol Trinatriumcitrat.2H₂O enthielt. Das erhaltene Filtrat wurde gefriergetrocknet und durch eine einstündige Dampfbehandlung bei 80° C ± 5° C und 1375 ± 35 mbar virusinaktiviert.

Das lyophilisierte virusinaktivierte Material wurde sodann in einer sterilen isotonen NaCl-Lösung gelöst und mittels Ionenaustauschchromatographie an Q-Sepharose wurden etwaig vorhandene Antikörper bzw. Serumamyloid P entfernt. Die gereinigte Lösung wurde durch einen weiteren Ultrafiltrations- und Diafiltrationsschritt konzentriert. Danach wurden zur erhaltenen Lösung pro Liter 10 g Albumin, 150 mMol NaCl und 15 mMol Trinatriumcitrat zugegeben. Der pH-Wert der Lösung betrug 7,5.

Maus-Immunglobulin sowie die Faktoren II, VII, IX und X konnten nicht nachgewiesen werden. Anschließend wurde die Lösung sterilfiltriert, abgefüllt und lyophilisiert. Die spezifische Aktivität betrug 14 Einheiten Protein C/mg. Eine Einheit Protein C Aktivität ist definiert als die Protein-C-Aktivität in 1 ml Normalplasma und wird gegen den ersten internationalen Standard von Protein C kalibriert. Als Aktivitätstest wurde ein amidolytischer Test verwendet, wobei das Protein C mittels Protac (Fa. Pentapharm) aktiviert wurde.

### Aktivierung von Protein C mit Plasmin

Protein C wurde in einer physiologischen Kochsalzlösung zu einer Lösung von 400 µg/ml gelöst und 10 E/ml Hirudin (Fa. Sigma) zugesetzt. Eine kommerziell erhältliche Plasminpräparation (Fa. Kabi) wurde in einem 0,4 %igen Natriumcitrat/0,7 %igen Natriumchlorid-Puffer gelöst und zu fünf Lösungen mit 0,01, 0,025, 0,05, 0,1 und 0,5 caseinolytischen Plasmineinheiten (PM) pro ml verdünnt.

Zur Aktivierung des Protein C wurden fünf Proben zu je 100 µl Protein C-Hirudin-Lösung mit 100 µl der obigen Plasmin-Lösungen gemischt. Nach 1, 5, 10, 30 und 60 Minuten Inkubation bei 37°C wurde die Plasminreaktion durch Zugabe von 100 µl Atheplex-Lösung (Antithrombin III-Heparin-Komplex, hergestellt nach EP-B - 0 129 534, 23 E Antithrombin III/ml und 150 E Heparin/ml 0,9 %iger NaCl-Lösung) gestoppt. Die Aktivität des aktivierten Protein C wurde amidolytisch mittels eines spezifischen chromogenen Substrats (S 2366, Fa. Kabi) gemessen. Die Ergebnisse sind aus dem Diagramm ersichtlich, wobei auf der Abszisse die Zeit in Minuten und auf der Ordinate die Aktivität (dE/min S 2366) eingetragen ist. Das Diagramm zeigt, daß die Aktivierung von Protein C mittels Plasmin sowohl zeit- als auch dosisabhängig ist. Das Verhältnis von Protein C zu Plasmin (µg/CU) betrug während der Reaktion 40.000 : 1, 16.000 : 1, 8.000 : 1, 4.000 : 1 bzw. 800 : 1.

Es hat sich weiters gezeigt, daß Plasmin nicht nur Protein C aktiviert, sondern bei längerer Einwirkung zu einem Abbau des aktivierten Protein C führt. Das gilt insbesondere bei einem Protein C/Plasmin-Verhältnis von weniger als 80 µg/CU. Bei einem Protein C/Plasmin-Verhältnis von 8 µg/CU beispielsweise führt eine Inkubation von 60 min zu einem vollständigen Abbau des aktivierten Protein C.

### Immunoblotting von aktiviertem Protein C

Immunoblotting wurde von Protein C, welches mit Plasmin aktiviert wurde, sowie von einem thrombinaktivierten Protein C und einem Protein C, aktiviert mit Protac (Fa. Pentapharm) durchgeführt. Es hat sich gezeigt, daß aktiviertes Protein C - unabhängig von der Art des Aktivierens - stets das gleiche Immunoblot ergibt.

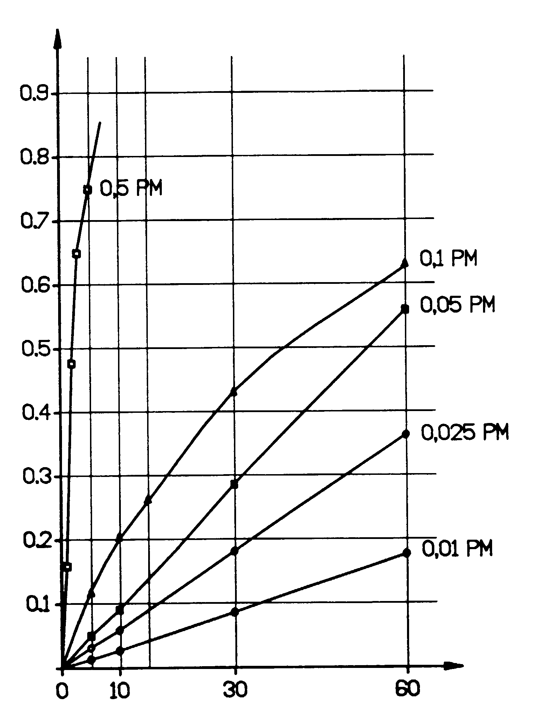

## Patentansprüche

1. Verfahren zur Herstellung von aktiviertem Protein C, wobei Protein C mit einer Protease behandelt wird, dadurch gekennzeichnet, daß als Protease Plasmin eingesetzt wird.

2. Verfahren nach Anspruch 1, dadurch gekennzeichnet, daß die Aktivierung bei einem Protein C/Plasmin-Verhältnis zwischen 80 und 40.000 µg/CU, vorzugsweise zwischen 4.000 und 40.000 µg/CU durchgeführt wird.

3. Verfahren nach Anspruch 1 oder Anspruch 2, dadurch gekennzeichnet, daß die Aktivierung in Gegenwart von Ca²⁺-Ionen durchgeführt wird.

4. Verfahren nach einem oder mehreren der Ansprüche 1 bis 3, dadurch gekennzeichnet, daß die Aktivierung durch Zugabe eines Plasmin-Inhibitors gestoppt wird.

## Claims

1. A method of preparing activated protein C, wherein protein C is treated with a protease, characterised in that plasmin is used as the protease.

2. A method according to claim 1, characterised in that the activation is carried out at a protein C/plasmin ratio of between 80 and 40,000 µg/CU, preferably between 4,000 and 40,000 µg/CU.

3. A method according to claim 1 or claim 2, characterised in that activation is carried out in the presence of Ca²⁺ ions.

4. A method according to one or several of claims 1 to 3, characterised in that activation is stopped by adding a plasmin inhibitor.

## Revendications

1. Procédé de préparation de protéine C activée, la protéine C étant traitée par une protéase, caractérisé en ce qu'on utilise de la plasmine comme protéase.

2. Procédé selon la revendication 1, caractérisé en ce qu'on procède à l'activation pour un rapport protéine C/plasmine compris entre 80 et 40 µg/CU, de préférence entre 4 000 et 40 000 µg/CU.

3. Procédé selon la revendication 1 ou la revendication 2, caractérisé en ce que l'activation s'effectue en présence d'ions Ca²⁺.

4. Procédé selon une ou plusieurs des revendications 1 à 3, caractérisé en ce que l'activation est arrêtée par addition d'un inhibiteur de la plasmine.
